# EUROPÄISCHE PATENTSCHRIFT

(11) **EP 0 376 122 B1**
(45) Veröffentlichungstag und Bekanntmachung des Hinweises auf die Patenterteilung: **06.04.1994**
(21) Anmeldenummer: 89123437.9
(22) Anmeldetag: 19.12.1989
(51) Int. Cl.: C07D 201/08

(54) **Verfahren zur Herstellung von Caprolactam**
Process for the preparation of caprolactam
Procédé de préparation de caprolactame

(30) Priorität: 24.12.1988 DE 3843793
(43) Veröffentlichungstag der Anmeldung: 04.07.1990
(73) Patentinhaber: BASF Aktiengesellschaft, 67063 Ludwigshafen (DE)
(72) Erfinder: Merger, Franz, Dr., D-6710 Frankenthal (DE); Fischer, Rolf, Dr., D-6900 Heidelberg (DE); Priester, Claus-Ulrich, Dr., D-6700 Ludwigshafen (DE)

(56) Entgegenhaltungen:
- EP-A- 0 271 815
- EP-A- 0 271 817
- DE-A- 2 249 993
- DE-A- 3 602 375
- DE-A- 3 602 376

## Beschreibung

Gegenstand der Erfindung ist ein Verfahren zur Herstellung von Caprolactam aus 6-Aminocapronsäureestern.

DE-A- 3602376 und DE-A- 3602375 betreffen Verfahren zur Herstellung von Caprolaktam aus 5-Formylvalerionsäureestern
In der DE-A- 22 49 993 wird ein Verfahren beschrieben, bei dem man 6-Aminocapronsäureester in Gegenwart von Wasser bei einer Temperatur von 250 bis 350°C zu Caprolactam umsetzt. Das Verfahren hat jedoch den Nachteil, daß, wie aus Industrial Engineering Chem. Proc. Design Development, Band 17 (1978), Seite 11, bekannt ist, diese Umsetzung über 6-Aminocapronsäure verläuft und daher, wie aus Seite 15 ersichtlich, die Cyclisierung bei niedrigen Konzentrationen durchgeführt werden soll, um die Bildung von Oligomeren zu unterdrücken. Dies hat zur Folge, daß sich die Isolierung des Caprolactams aus den verdünnten wäßrigen Lösungen technisch aufwendig gestaltet.

In der DE-A 36 43 010 (EP-A- 0271815) wird die Cyclisierung von 6-Aminocapronsäureestern zu Caprolactam in aromatischen Kohlenwasserstoffen mit einem Siedepunkt von 80 bis 240°C als Reaktionsmedium bei einer Temperatur von 100 bis 320°C beschrieben. Die Reaktion bedarf jedoch Verweilzeiten bis zu 15 h. Die Reaktionsgeschwindigkeit und die Raum-Zeit-Ausbeuten sind so niedrig, daß sie für die technische Durchführung des Verfahrens verbesserungsbedürftig sind.

Auch nach dem in der DE-A- 36 43 011 (EP-A- 0271817) beschriebenen Verfahren zur Herstellung von Caprolactam durch Cyclisierung von 6-Aminocapronsäureestern in einem unter Reaktionsbedingungen inerten flüssigen Reaktionsmedium mit einem Siedepunkt über dem des Caprolactams, bei einer Temperatur von 150 bis 350°C, wobei die Reaktionsprodukte Caprolactam und Alkanol kontinuierlich aus dem Reaktionsgemisch entfernt werden in dem Maße wie sie entstehen, erzielt man Raum-Zeit-Ausbeuten, die verbesserungsbedürftig sind.

Es war deshalb die technische Aufgabe gestellt, ein Verfahren zur Herstellung von Caprolactam, ausgehend von 6-Aminocapronsäureestern zur Verfügung zu stellen, das mit hoher Selektivität und hohem Umsatz sowie in technisch befriedigender Raum-Zeit-Ausbeute verläuft und die Bildung von Nebenprodukten minimiert.

Diese Aufgabe wird gelöst, in einem Verfahren zur Herstellung von Caprolactam durch Erhitzen von 6-Aminocapronsäureestern in Gegenwart von einem unter Reaktionsbedingungen inerten flüssigen aromatischen Kohlenwasserstoff mit einem Siedepunkt von 80 bis 240°C unter erhöhtem Druck in flüssiger Phase, wobei man zusätzlich Wasser mitverwendet, eine Temperatur von 230°C bis 350°C einhält und Caprolactam aus dem Reaktionsmedium gewinnt.

Das neue Verfahren hat den Vorteil, daß es mit verbesserten Raum-Zeit-Ausbeuten verläuft und hohe Umsätze und Selektivitäten erzielt werden. Darüber hinaus hat das neue Verfahren den Vorteil, daß trotz Mitverwendung von Wasser die Bildung von Nebenprodukten vermieden wird.

Bevorzugte Ausgangsstoffe sind 6-Aminocapronsäureester von Alkanolen mit 1 bis 10 Kohlenstoffatomen, Cycloalkanolen mit 5 bis 8 Kohlenstoffatomen oder Aralkanolen mit 7 bis 10 Kohlenstoffatomen. Geeignete Ausgangsstoffe sind beispielsweise 6-Aminocapronsäuremethylester, -ethylester, -i-propylester, -n-propylester, -cyclohexylester oder -benzylester. Besonders bevorzugt sind 6-Aminocapronsäurealkylester, insbesondere 6-Aminocapronsäure-C₁-C₄-alkylester. Besondere technische Bedeutung haben 6-Aminocapronsäuremethyl-oder -ethylester erlangt.

Erfindungsgemäß verwendet man als Reaktionsmedium unter Reaktionsbedingungen inerte flüssige aromatische Kohlenwasserstoffe mit einem Siedepunkt von 80 bis 240°C, insbesondere mit einem Siedepunkt von 110 bis 200°C. Bevorzugte aromatische Kohlenwasserstoffe sind Alkylbenzole, insbesondere solche, die 1 bis 3 Alkylgruppen mit bis zu 6 Kohlenstoffatomen enthalten. Besonders bevorzugt sind Alkylbenzole mit 1 bis 3 Alkylresten mit insgesamt bis zu 4 Kohlenstoffatomen. Geeignete aromatische Kohlenwasserstoffe sind beispielsweise Benzol, Toluol, Xylole, Ethylbenzol, Diethylbenzol, Trimethylbenzol, Isopropylbenzol, Propyl- oder Diisopropylbenzol. Besonders bevorzugt sind Toluol und Xylole.

In der Regel verwendet man je kg Aminocapronsäureester 2 bis 20 kg, insbesondere 4 bis 15 kg, aromatische Kohlenwasserstoffe. Besonders bevorzugt ist ein Bereich von 5 bis 12 kg aromatischen Kohlenwasserstoffen je kg 6-Aminocapronsäureester.

Für die Umsetzung wird erfindungsgemäß zusätzlich Wasser mitverwendet. In der Regel wendet man je Mol 6-Aminocapronsäureester 1 bis 10 Mol Wasser an. Besonders bewährt hat es sich, wenn man je mol 6-Aminocapronsäureester 1,5 bis 6 mol Wasser, insbesondere 2 bis 4 mol Wasser mitverwendet.

Die Umsetzung wird bei einer Temperatur von 230 bis 350°C, vorteilhaft 260 bis 340°C durchgeführt.

Die Temperaturbedingungen und Druckbedingungen werden so gewählt, daß das Reaktionsgemisch stets in flüssigem Zustand vorliegt. Vorteilhaft stellt man einen Druck von 30 bis 200 bar, insbesondere 40 bis 110 bar ein.

Vorteilhaft hält man bei der Umsetzung eine Verweilzeit von 5 bis 60 min, insbesondere von 7 bis 45 min ein. Besonders bewährt hat sich eine Verweilzeit von 10 bis 20 min.

Die Mischung aus 6-Aminocapronsäureester Wasser und aromatischen Kohlenwasserstoffen wird auf die angegebene Temperatur erhitzt, wobei man die Druck- und Temperaturbedingungen so wählt, daß das Reaktionsgemisch im flüssigen Zustand vorliegt. Die Umsetzung kann diskontinuierlich, z.B. in Druckbehältern durchgeführt werden. Vorzugsweise führt man die Umsetzung kontinuierlich, z. B. in Druckbehälterkaskaden, z.B. in 2 bis 4 hintereinandergeschalteten Druckbehältern durch. Besonders bewährt hat es sich, wenn man die Umsetzung in einer rohrförmigen Reaktionszone durchführt. Geeignete Reaktionszonen haben beispielsweise ein Verhältnis von Länge zu Durchmesser von 100 bis 10 000 : 1. Hierbei hat es sich bewährt, wenn man die Ausgangsstoffe, z.B. eine Lösung von 6-Aminocapronsäureestern in aromatischen Kohlenwasserstoffen und Wasser emulgiert, vorteilhaft bei einer Temperatur von -10 bis +20°C und dann die Emulsion in den Rohrreaktor einbringt.

Aus der so erhaltenen Lösung von Caprolactam in aromatischen Kohlenwasserstoffen wird Caprolactam im allgemeinen durch fraktionierende Destillation gewonnen und die aromatischen Kohlenwasserstoffe wieder zurückgeführt. Nach einer bevorzugten Arbeitsweise wird Caprolactam aus den aromatischen Kohlenwasserstoffen mit Wasser extrahiert. Vorteilhaft wird die Extraktion im Gegenstrom in bekannten Vorrichtungen, z. B. Mixer-Settler, Rührscheibenkolonnen oder Siebbodenkolonnen mit oder ohne Pulsation durchgeführt. Vorteilhaft hält man bei der Extraktion eine Temperatur von 20 bis 80°C ein. Hierbei hat es sich bewährt, wenn ein Teilstrom der aromatischen Kohlenwasserstoffe vor der Wiederverwendung durch Destillation gereinigt wird.

Caprolactam eignet sich zur Herstellung von Polycaprolactam, einem wichtigen Faserrohstoff.

Das Verfahren nach der Erfindung sei an folgenden Beispielen veranschaulicht.

### Beispiel 1

Durch einen 70 ml Rohrreaktor mit einem Innendurchmesser von 2,2 mm und einer Länge von 18,4 m werden bei einem Druck von 100 bar, einer Temperatur von 270°C und einer Verweilzeit von 15,8 Minuten stündlich 251,0 ml (220,0 g) einer Lösung von 8,80 Gew.-% (19,36 g, 0,134 mol) 6-Aminocapronsäuremethylester und 0,16 Gew.-% Caprolactam (0,36 g, 0,003 mol) in o-Xylol und 14,4 ml (0,80 mol) Wasser gepumpt. Die Reaktionslösung wird über ein überströmventil auf Normaldruck entspannt und die Zusammensetzung des Austrages nach Homogenisierung mit Methanol durch quantitative Gaschromatographie analysiert. Neben 0,57 g unumgesetztem 6-Aminocapronsäuremethylester (Umsatz: 97,1 %) wurden 14,50 g (Selektivität: 96,6 %) Caprolactam erhalten.

### Beispiel 2

Durch einen 70 ml Rohrreaktor mit einem Innendurchmesser von 2,2 mm und einer Länge von 18,4 m werden bei einem Druck von 100 bar, einer Temperatur von 270°C und einer Verweilzeit von 15,2 Minuten stündlich 265,6 ml (232,8 g) einer Lösung von 8,96 Gew.-% (20,86 g, 0,144 mol) 6-Aminocapronsäuremethylester und 0,17 Gew.-% Caprolactam (0,40 g, 0,004 mol) in o-Xylol und 10,8 ml (0,60 mol) Wasser gepumpt. Die Reaktionslösung wird über ein überströmventil auf Normaldruck entspannt und die Zusammensetzung des Austrages nach Homogenisierung mit Methanol durch quantitative Gaschromatographie analysiert. Neben 1,03 g unumgesetztem 6-Aminocapronsäuremethylester (Umsatz: 95,1 %) wurden 14,33 g (Selektivität: 90,1 %) Caprolactam erhalten.

### Beispiel 3

Durch einen 70 ml Rohrreaktor mit einem Innendurchmesser von 2,2 mm und einer Länge von 18,4 m werden bei einem Druck von 100 bar, einer Temperatur von 270°C und einer Verweilzeit von 18,0 Minuten stündlich 224,0 ml (196,4 g) einer Lösung von 8,94 Gew.-% (17,56 g, 0,121 mol) 6-Aminocapronsäuremethylester und 0,17 Gew.-% Caprolactam (0,33 g, 0,003 mol) in o-Xylol und 9,6 ml (0,53 mol) Wasser gepumpt. Die Reaktionslösung wird über ein überströmventil auf Normaldruck entspannt und die Zusammensetzung des Austrages nach Homogenisierung mit Methanol durch quantitative Gaschromatographie analysiert. Neben 0,46 g unumgesetztem 6-Aminocapronsäuremethylester (Umsatz: 97,4 %) wurden 12,45 g (Selektivität: 90,9 %) Caprolactam erhalten.

### Beispiel 4

Durch einen 70 ml Rohrreaktor mit einem Innendurchmesser von 2,2 mm und einer Länge von 18,4 m werden bei einem Druck von 100 bar, einer Temperatur von 270°C und einer Verweilzeit von 15,7 Minuten stündlich 260,0 ml (228,9 g) einer Lösung von 8,53 Gew.-% (19,53 g, 0,135 mol) 6-Aminocapronsäuremethylester und 0,17 Gew.-% Caprolactam (0,39 g, 0,003 mol) in o-Xylol und 8,3 ml (8,1 g) einer Lösung von 5,19 Gew.-% (0,42 g) 6-Aminocapronsäuremethylester und 3,21 Gew.-% Caprolactam (0,26 g) in Wasser gepumpt. Die Reaktionslösung wird über ein überströmventil auf Normaldruck entspannt und anschließend der Austrag von einer Stunde am Rotationsverdampfer im Vakuum eingeengt. Der Rückstand enthält nach quantitativer gaschromatographischer Analyse 0,61 g 6-Aminocapronsäuremethylester (Umsatz: 96,9 %) und 14,95 g (Selektivität: 94,9 %) Caprolactam.

### Beispiel 5

Durch einen 70 ml Rohrreaktor mit einem Innendurchmesser von 2,2 mm und einer Länge von 18,4 m werden bei einem Druck von 100 bar, einer Temperatur von 270°C und einer Verweilzeit von 15,4 Minuten stündlich 264,7 ml (234,1 g) einer Lösung von 8,50 Gew.-% (19,90 g, 0,137 mol) 6-Aminocapronsäuremethylester und 0,16 Gew.-% Caprolactam (0,37 g, 0,003 mol) in o-Xylol und 8,9 ml (8,9 g) einer Lösung von 7,53 Gew.-% (0,67 g) 6-Aminocapronsäuremethylester und 4,61 Gew.-% Caprolactam (0,41 g) in Wasser gepumpt. Die Reaktionslösung wird über ein überströmventil auf Normaldruck entspannt und die Zusammensetzung des Austrages nach Homogenisierung mit Methanol durch quantitative Gaschromatographie analysiert. Neben 0,2 g unumgesetztem 6-Aminocapronsäuremethylester (Umsatz: 98,0 %) wurden 14,74 g (Selektivität: 88,9 %) Caprolactam erhalten.

### Beispiel 6

367,1 g einer Lösung von 9,31 Gew.-% (34,19 g, 0,236 mol) 6-Aminocapronsäuremethylester, 0,32 Gew.-% (1,19 g, 0,011 mol) Caprolactam in o-Xylol und 15,9 g (0,88 mol) Wasser werden in einem auf 0°C thermostatisierten Doppelmantelkolben emulgiert und daraus bei einem Druck von 70 bar, einer Temperatur von 300°C und einer Verweilzeit von 10 Minuten durch einen 70-ml-Rohrreaktor gepumpt. Anschließend wird der Reaktor mit 100 ml Xylol gespült und die gesammelten Austräge am Rotationsverdampfer i. Vak. eingeengt. Es verbleiben 36,9 g Produkt, das nach quantitativer gaschromatographischer Analyse 6.37 % 6-Aminocapronsäuremethylester (2,35 g, Umsatz: 93,1 %) und 64,88 % (23,94 g, Selektivität: 91,7 %) Caprolactam enthält.

### Beispiel 7

463,0 g einer Lösung von 9,32 Gew.-% (43,15 g, 0,298 mol) 6-Aminocapronsäuremethylester, 0,27 Gew.-% (1,26 g, 0,011 mol) Caprolactam in o-Xylol und 19,7 g (1,09 mol) Wasser werden in einem auf 0°C thermostatisierten Doppelmantelkolben emulgiert und daraus bei einem Druck von 70 bar, einer Temperatur von 330°C und einer Verweilzeit von 10 Minuten durch einen 70 ml Rohrreaktor gepumpt. Anschließend wird der Reaktor mit 100 ml Xylol gespült und die gesammelten Austräge am Rotationsverdampfer i. Vak. eingeengt. Es verbleiben 47,8 g Produkt, das nach quantitativer gaschromatographischer Analyse 3,99 % 6-Aminocapronsäuremethylester (1,91 g, Umsatz: 95,6 %) und 63,23 % (30,22 g, Selektivität: 90,1 %) Caprolactam enthält.

### Beispiel 8

669,5 g einer Lösung von 10,26 Gew.-% (68,70 g, 0,474 mol) 6-Aminocapronsäuremethylester, 0,32 Gew.-% (2,17 g, 0,019 mol) Caprolactam in o-Xylol und 10,0 g (0,56 mol) Wasser werden in einem auf 0°C thermostatisierten Doppelmantelkolben emulgiert und daraus bei einem Druck von 70 bar, einer Temperatur von 270°C und einer Verweilzeit von 11,7 Minuten durch einen 70-ml-Rohrreaktor gepumpt. Anschließend wird der Reaktor mit 70 ml Xylol gespült und die gesammelten Austräge am Rotationsverdampfer i.Vak. eingeengt. Es verbleiben 72,3 g Produkt, das nach quantitativer gaschromatographischer Analyse 5,80 % 6-Aminocapronsäuremethylester (4,20 g, Umsatz: 93,9 %) und nach quantitativer HPLC 77,7 % (49,01 g, Selektivität: 93,2 %) Caprolactam enthält.

### Beispiel 9

591,1 g einer Lösung von 22,27 Gew.-% (131,62 g, 0,908 mol) 6-Aminocapronsäuremethylester, 0,65 Gew.-% (3,84 g, 0,033 mol) Caprolactam in o-Xylol und 17,7 g (0,98 mol) Wasser werden in einem auf 0°C thermostatisierten Doppelmantelkolben emulgiert und daraus bei einem Druck von 70 bar, einer Temperatur von 270°C und einer Verweilzeit von 11,7 Minuten durch einen 70-ml- Rohrreaktor gepumpt. Anschließend wird der Reaktor mit 70 ml Xylol gespült und die gesammelten Austräge am Rotationsverdampfer i. Vak. eingeengt. Es verbleiben 104,2 g Produkt, das nach quantitativer gaschromatographischer Analyse 0,34 % 6-Aminocapronsäuremethylester (0,35 g, Umsatz: 99,7 %) und 90,06 % (93,84 g, Selektivität: 88,0 %) Caprolactam enthält.

### Beispiel 10

416,1 g einer Lösung von 19,95 Gew.-% (83,00 g, 0,572 mol) 6-Aminocapronsäuremethylester, 0,53 Gew.-% (2,19 g, 0,019 mol) Caprolactam in o-Xylol und 22,2 g (1,23 mol) Wasser werden in einem auf 0°C thermostatisierten Doppelmantelkolben emulgiert und daraus bei einem Druck von 70 bar, einer Temperatur von 270°C und einer Verweilzeit von 8,4 Minuten durch einen 70-ml-Rohrreaktor gepumpt. Der Austrag wird Methanol homogenisiert. Nach quantitativer gaschromatographischer Analyse wurden 61,8 g Caprolactam (Ausbeute: 92,2 %) gebildet.

### Beispiel 11

Eine Mischung aus 9,97 Gew.-% 6-Aminocapronsäuremethylester, 0,09 Gew.-% Caprolactam, 1,3 Gew.-% Wasser und 88,64 Gew.-% o-Xylol wird in einem auf 0°C thermostatisierten Doppelmantelkolben emulgiert und daraus bei einem Druck von 40 bar, einer Temperatur von 270°C und Verweilzeiten von 9,5 bis 43 Minuten durch einen 70-ml-Rohrreaktor gepumpt. Die Austräge werden mit Methanol homogenisiert und durch quantitative Gaschromatographie analysiert.

| Versuch | Verweilzeit [min] | Umsatz [%] | Selektivität [%] |
|---|---|---|---|
| A | 9,5 | 91,9 | 94,2 |
| B | 14 | 94,5 | 91,1 |
| C | 43 | 100 | 97,4 |

### Vergleichsbeispiel 1

Eine Lösung von 9,55 Gew.-% 6-Aminocapronsäuremethylester und 0,27 Gew.-% Caprolactam in o-Xylol wird bei einem Druck von 40 bar, einer Temperatur von 270°C und Verweilzeiten von 11 bis 44 Minuten durch einen 70-ml-Rohrreaktor gepumpt. Die Austräge werden durch quantitative Gaschromatographie analysiert.

| Versuch | Verweilzeit [min] | Umsatz [%] | Selektivität [%] |
|---|---|---|---|
| A | 11 | 49,8 | 80,9 |
| B | 25 | 77,2 | 80,7 |
| C | 33,5 | 91,9 | 81,7 |
| D | 44 | 98,7 | 82,9 |

### Vergleichsbeispiel 2

Eine Lösung von 15 g (0,0943 mol) 6-Aminocapronsäureethylester in 45 ml Wasser wurde in einem 0,25-l-Schüttelautoklaven innerhalb von 3 Stunden von 15 auf 270°C aufgeheizt und anschließend 1 Stunde bei dieser Temperatur unter Eigendruck (52 bis 65 bar) geschüttelt. Nach Abkühlen auf Raumtemperatur, Extraktion mit Trichlormethan (5 mol 20 ml), Trocknen über Natriumsulfat und Einengen im Vakuum wurden 8 g Roh-Caprolactam (Reinheit lt. GC: 94 %) mit Schmelzpunkt 66 bis 67°C erhalten entsprechend einer Ausbeute von 70,5 %.

### Vergleichsbeispiel 3

Eine Lösung von 20 g (0,126 mol) 6-Aminocapronsäureethylester in 60 ml Wasser wurde in einem 0,25-l-Schüttelautoklaven innerhalb von 5,5 Stunden von 20 auf 300°C aufgeheizt und anschließend 15 Minuten bei dieser Temperatur unter Eigendruck (80 bis 85 bar) geschüttelt. Nach Abkühlen auf Raumtemperatur, Extraktion mit Trichlormethan (5 mal 50 ml), Trocknen über Natriumsulfat und Einengen im Vakuum wurden 11,3 g Roh-Caprolactam mit Schmelzpunkt 65 bis 68°C erhalten entsprechend einer Ausbeute von 79,4 %.

### Vergleichsbeispiel 4

Eine Lösung von 20 g (0,126 mol) 6-Aminocapronsäureethylester in 60 ml Wasser wurde in einem 0,25-l-Schüttelautoklaven innerhalb von 5,5 Stunden auf 300°C aufgeheizt und anschließend 1 Stunde bei dieser Temperatur unter Eigendruck (60 bis 90 bar) geschüttelt. Nach Abkühlen auf Raumtemperatur, Extraktion mit Trichlormethan )7 mal 20 ml), Trocknen über Natriumsulfat und Einengen im Vakuum wurden 11,9 g Roh-Caprolactam mit Schmelzpunkt 64 bis 66°C isoliert entsprechend einer Ausbeute von 83,6 %.

### Vergleichsbeispiel 5

Eine Lösung von 30,8 g (0,212 mol) 6-Aminocapronsäuremethylester und 4,8 g (0,042 mol) Caprolactam in 112,5 g Wasser wurde in einem 375-ml-Rührautoklaven innerhalb von einer Stunde auf 300°C aufgeheizt, wobei sich ein Druck von 80 bar einstellte. Nach dieser Zeit enthielt die Reaktionslösung nach quantitativer gaschromatographischer Analyse 15,38 Gew.-% Caprolactam (entsprechend einer Ausbeute von 76 %, bezogen auf 6-Aminocapronsäuremethylester) und daneben 0,57 % N-Methyl-caprolactam.

### Vergleichsbeispiel 6

Durch einen 40-ml-Rohrreaktor wurde bei einer Temperatur von 300°C, einem Druck von 100 bar und einer Verweilzeit von 15 Minuten eine Lösung von 18,28 % 6-Aminocapronsäuremethylester und 5,75 % Caprolactam in Wasser gepumt. Der Reaktionsaustrag enthielt nach quantitativer gaschromatographischer Analyse 15,09 Gew.-% Caprolactam entsprechend einer Ausbeute von 65,6 %, bezogen auf 6-Aminocapronsäuremethylester. Als Nebenprodukt entstand in 5,8 % Ausbeute N-Methyl-caprolactam.

### Vergleichsbeispiel 7

Durch einen 40-ml-Rohrreaktor wurde bei einer Temperatur von 270°C, einem Druck von 100 bar und einer Verweilzeit von 4,2 Minuten eine Lösung von 15,7 % 6-Aminocapronsäuremethylester und 8,2 % Caprolactam in Wasser gepumpt. Der Reaktionsaustrag enthielt nach quantitativer gaschromatographischer Analyse 14,98 Gew.-% Caprolactam, entsprechend einer Ausbeute von 55,4 %, bezogen auf 6-Aminocapronsäuremethylester. Als Nebenprodukt hatten sich 4,5 % N-Methyl-caprolactam gebildet.

## Patentansprüche

1. Verfahren zur Herstellung von Caprolactam durch Erhitzen von 6-Aminocapronsäureestern in Gegenwart von einem unter Reaktionsbedingungen inerten flüssigen aromatischen Kohlenwasserstoff mit einem Siedepunkt von 80 bis 240°C als Reaktionsmedium unter erhöhtem Druck in flüssiger Phase, dadurch gekennzeichnet, daß man zusätzlich Wasser mitverwendet, eine Temperatur von 230 bis 350°C einhält und Caprolactam aus dem Reaktionsgemisch gewinnt.

2. Verfahren nach Anspruch 1, dadurch gekennzeichnet, daß man eine Temperatur von 260 bis 340°C einhält.

3. Verfahren nach den Ansprüchen 1 bis 2, dadurch gekennzeichnet, daß man einen Druck von 30 bis 200 bar einhält.

4. Verfahren nach den Ansprüchen 1 bis 3, dadurch gekennzeichnet,' daß man je mol 6-Aminocapronsäureester 1 bis 10 mol Wasser mitverwendet.

5. Verfahren nach den Ansprüchen 1 bis 4, dadurch gekennzeichnet, daß man eine Verweilzeit von 5 bis 60 Minuten einhält.

6. Verfahren nach den Ansprüchen 1 bis 5, dadurch gekennzeichnet, daß man Alkylbenzole mit einem Siedepunkt von 110 bis 200°C verwendet.

7. Verfahren nach den Ansprüchen 1 bis 6, dadurch gekennzeichnet, daß man Toluol oder Xylole verwendet.

8. Verfahren nach den Ansprüchen 1 bis 7, dadurch gekennzeichnet, daß man die Umsetzung in einer rohrförmigen Reaktionszone durchführt.

9. Verfahren nach den Ansprüchen 1 bis 8, dadurch gekennzeichnet, daß man vor der Umsetzung 6-Aminocapronsäureester, aromatische Kohlenwasserstoffe und Wasser bei einer Temperatur von -10 bis +20 °C ermulgiert.

10. Verfahren nach den Ansprüchen 1 bis 9, dadurch gekennzeichnet, daß man 6-Aminocapronsäure-C₁-C₄-alkylester verwendet.

## Claims

1. A process for the preparation of caprolactam by heating 6-aminocaproic esters in the presence of a liquid aromatic hydrocarbon which is inert under the reaction conditions and has a boiling point of 80 to 240°C as reaction medium under elevated pressure in liquid phase, wherein water is additionally used, a temperature of 230 to 350°C is maintained, and caprolactam is isolated from the reaction mixture.

2. A process as claimed in claim 1, wherein a temperature of 260 to 340°C is maintained.

3. A process as claimed in claims 1 to 2, wherein a pressure of 30 to 200 bar is maintained.

4. A process as claimed in claims 1 to 3, wherein 1 to 10 mol of water are additionally used per mol of 6-aminocaproic ester.

5. A process as claimed in claims 1 to 4, wherein a holdup time of 5 to 60 minutes is maintained.

6. A process as claimed in claims 1 to 5, wherein alkylbenzenes with a boiling point of 110 to 200°C are used.

7. A process as claimed in claims 1 to 6, wherein toluene or xylenes are used.

8. A process as claimed in claims 1 to 7, wherein the reaction is carried out in a tubular reaction zone.

9. A process as claimed in claims 1 to 8, wherein 6-aminocaproic ester, aromatic hydrocarbons and water are emulsified at a temperature of -10 to +20°C before the reaction.

10. A process as claimed in claims 1 to 9, wherein C₁-C₄-alkyl 6-aminocaproates are used.

## Revendications

1. Procédé de préparation de caprolactame par chauffage d'esters d'acide 6-aminocaproïque en présence d'un hydrocarbure aromatique liquide inerte dans les conditions de la réaction, ayant un point d'ébullition compris entre 80 et 240°C, en tant que milieu réactionnel, sous pression élevée en phase liquide, caractérisé en ce que l'on utilise de plus de l'eau, en ce que l'on fixe une température de 230 à 350°C et en ce que l'on récupère du caprolactame à partir du mélange réactionnel.

2. Procédé selon la revendication 1, caractérisé en ce que l'on fixe une température de 260 à 340°C.

3. Procédé selon la revendication 1 ou 2, caractérisé en ce que l'on fixe une pression de 30 à 200 bar.

4. Procédé selon l'une des revendications 1 à 3, caractérisé en ce que l'on utilise 1 à 10 moles d'eau par mole d'ester d'acide 6-aminocaproïque.

5. Procédé selon l'une quelconque des revendications 1 à 4, caractérisé en ce que l'on met en oeuvre un temps de séjour de 5 à 60 minutes.

6. Procédé selon l'une quelconque des revendications 1 à 5, caractérisé en ce que l'on utilise des alkylbenzènes ayant un point d'ébullition compris entre 110 et 200°C.

7. Procédé selon l'une quelconque des revendications 1 à 6, caractérisé en ce que l'on utilise du toluène ou des xylènes.

8. Procédé selon l'une quelconque des revendications 1 à 7, caractérisé en ce que l'on effectue la réaction dans une zone réactionnelle tubulaire.

9. Procédé selon l'une quelconque des revendications 1 à 8, caractérisé en ce que l'on réalise, avant la réaction, une émulsion d'ester d'acide 6-aminocaproïque, d'hydrocarbures aromatiques et d'eau à une température comprise entre -10 et +20°C.

10. Procédé selon l'une quelconque des revendications 1 à 9, caractérisé en ce que l'on utilise un 6-aminocaproate d'alkyle en C₁-C₄.
